# NEW EUROPEAN PATENT SPECIFICATION

(11) **EP 1 330 229 B2**
(45) Date of publication and mention of the opposition decision: **24.10.2012**
(45) Mention of the grant of the patent: 26.01.2005
(21) Application number: 01980525.8
(22) Date of filing: 26.10.2001
(51) Int. Cl.: A61K 8/39, A61K 8/49, A61K 8/89, A61Q 5/02

(54) **COMPOSITIONS COMPRISING HYDROPHOBIC SILICONE OILS AND ETHOXYLATED GLYCERIDES**
ZUSAMMENSETZUNGEN, DIE HYDROPHOBISCHE SILICONÖLE UND ETHOXYLIERTE GLYCERIDE ENTHALTEN
COMPOSITIONS COMPRENANT DES HUILES DE SILICONE HYDROPHOBES ET DE GLYCERIDES ETHOXYLES

(30) Priority: 30.10.2000 DE 10053725
(43) Date of publication of application: 30.07.2003
(73) Proprietor: Kao Corporation, Chuo-ku Tokyo (JP)
(72) Inventor: DENZER, Horst, 40219 Düsseldorf (DE); ABE, Hiroshi, E-08021 Barcelona (ES); PYTLIK, Monika, 47269 Duisburg (DE); JANSEN, Rosemarie, 46446 Emmerich/Elten (DE); BUHMANN, Andrea, 30163 Hannover (DE)
(74) Representative: HOFFMANN EITLE
(86) International application number: PCT/EP2001/012437
(87) International publication number: WO 2002/036087

(56) References cited:
- EP-A- 0 407 089
- EP-A- 0 529 883
- EP-A1- 0 440 542
- EP-A2- 0 998 906
- WO-A1-92/04882
- WO-A1-99/630032
- US- - 5 290 555
- US- - 5 759 983
- US-A- 5 104 645
- US-A- 6 015 574
- PATENT ABSTRACTS OF JAPAN vol. 1995, no. 01, 28 February 1995 (1995-02-28) & JP 06 293619 A (ASAHI CHEM IND CO LTD), 21 October 1994 (1994-10-21)

## Description

The present invention relates to optically transparent aqueous compositions containing hydrophobic silicone oil and ethoxylated glycerides, the compositions being suitable as a hair treatment composition such as a shampoo.

Due to their very low surface tension, the spreadability of silicone oils on most surfaces such as ceramics, textiles, paper, skin, and hair, is excellent. In the field of personal care products, silicone oils are used because of their hair and skin smoothing properties, hair gloss enhancing properties and skin feel improving (non-oily, silky skin feel) properties. For many decades they are therefore ingredients in hairsprays, conditioners, colorants and sun protecting creams. In cosmetic rinse-off products like shampoos they appeared in the 1980ies and could obtain a considerable market share in the early 1990ies in the so-called "two-in-one" shampoos. These shampoos contain emulsified silicone oils. Silicone oil emulsions, however, show problems with respect to compatibility and stability, they show a strong foaminess reducing effect and furthermore they are generally not transparent. This is why hydrophilic silicone polyethers have been introduced into the market. But apart from their generally higher price, the conditioning effect of hydrophilic silicone polyethers on skin and hair is generally much lower than of the hydrophobic silicone oils.

In view of these problems, attempts have been made to provide aqueous compositions containing hydrophobic silicone oil, the silicone oil being in a solubilized or micro-emulsified state.

US 6 013 683 describes a microemulsion containing 40 to 95 wt.% of a short chain linear siloxane and water, and 5 to 60 wt.% of non-ionic and/or cationic surfactants. However, the microemulsions disclosed in this patent are only transparent in a very narrow temperature range and easily become turbid when added to aqueous solutions.

EP 0 529 883 B1 discloses hair shampoo compositions containing sodium lauryl ether sulfate and cocoamido propyl betaine as surfactants and 1.0 wt.-% of silicone oil. The silicone oil was added as micro-emulsion prepared by an emulsion polymerization technique. Hence, EP 0 529 883 B1 does not disclose aqueous compositions containing silicone oil which may be easily prepared.

On the other hand, the inventors of the present invention previously published a method allowing the easy incorporation of silicone oil into shampoos (H. Denzer, R. Jansen, M. Reininghaus in "Parfumerie und Kosmetik"; 6/99, pages 18-20). However, the method only allowed for the incorporation of comparably low amounts of silicone oil such as 0.5 wt.-% when using wash-active matter within the range of 15-40 wt.-%. Higher amounts of hydrophobic silicone oil could only be solubilized by increasing the amount of wash-active matter which is, however, not acceptable for dermatological and environmental reasons as well as for price reasons.

In view of these drawbacks of the prior art, it is the object underlying the present invention to provide an easily preparable, optically transparent aqueous composition being suitable as hair shampoo containing an increased amount of hydrophobic silicone oil.

This object of the present invention is solved by the provision of an optically transparent aqueous composition comprising
(a) a hydrophobic silicone oil in an amount of 1-3 wt.-% with respect to the total weight of the composition;
(b) ethoxylated glycerides derived from carboxylic acids having 6 to 22 carbon atoms;
(c) an anionic surfactant;
wherein the weight ratio of component (b) to component (a) is in the range of 1:1 to 10:1; and wherein the total amount of the components (b) and (c) is in the range of 10-25 wt.-% with respect to the total weight of the composition.

A hydrophobic silicone oil is generally a silicone oil which is soluble in paraffinic oil at 25°C. Hydrophobic silicone oils to be used according to the present invention include both volatile and non-volatile silicone oils.

Specific examples include a cyclic methyl siloxane having the formula {(CH₃)₂SiO}ₓ in which x is 3-6, or short chain linear methyl siloxanes having the formula ((CH₃)₂SiO{(CH₃)₂SiO}_{y}Si(CH₃)₃ in which y is 0-5.

Some suitable cyclic methyl siloxanes are hexamethylcyclotrisiloxanes (D₃), a solid with a boiling point of 134°C and the formula {(Me₂)SiO}₃; octamethylcyclotetrasiloxane (D₄) with a boiling point of 176°C, a viscosity of 2.3 mm²/s, and the formula {(Me₂)SiO}₄; decamethylcyclopentasiloxane (D₅) (cyclomethicone) with a boiling point of 210°C, a viscosity of 3.87 mm²/s, and the formula {(Me₂)SiO}₅; and dodecamethylcyclohexasiloxane (D₆) with a boiling point of 245°C, a viscosity of 6.62 mm²/s and the formula {(Me₂)SiO}₆.

Some suitable short linear methyl siloxane are hexamethyldisiloxane (MM) with a boiling point of 100°C, viscosity of 0-65 mm²/s, and formula Me₃SiOMe₃; octamethyltrisiloxane (MDM) with a boiling point of 152°C, viscosity of 1.04 mm²/s, and formula Me₃SiOMe₂SiOsiMe₃; decamethyltetrasiloxane (MD₂M) with a boiling point of 194°C, viscosity of 1.53 mm²/s, and formula Me₃SiO(MeSiO)₂SiMe₃; dodecamethylpentasiloxane (MD₃M) with a boiling point of 229°C, viscosity of 2.06 mm₂/s, and formula Me₃SiO(Me₂SiO)₃SiMe₃; tetradecamethylhexasiloxane (MD₄M) with a boiling point of 245°C, viscosity of 2.63 mm2/s, and formula Me₃SiO(Me₂SiO)₄SiMe₃; and hexadecamethylheptasiloxane (MD₅M) with a boiling point of 270°C, viscosity.of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, and dimethiconol are also included.

The amount of the hydrophobic silicone oil is 1-3 wt.-%, preferably 1.5 wt.% to 3 wt.% with respect to the total weight of the composition.

The ethoxylated glycerides derived from carboxylic acids having 6 to 22 carbon atoms preferably include compounds of the following formula (I): 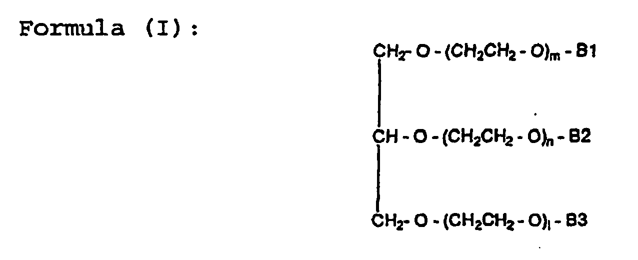 wherein each of m, n, and 1 independently represent a number from 0 to 40, the sum of m, n and 1 being in the range of 1 to 200, preferably 9 to 19; and B1, B2, and B3 independently represent H or an acyl residue having 6 to 22 carbon atoms, with the proviso that at least one of B1, B2 and B3 is an acyl residue having 6 to 22 carbon atoms.

The ethoxylated glyceride, component (b), is desirably used as a mixture of compounds of the above formula (I) comprising (i) compounds represented by the above formula (I), wherein each of B1, B2 and B3 independently represent an acyl group having 6 to 22 carbon atoms;
(ii) compounds represented by the above formula (I), wherein two of B1, B2 and B3 independently represent an acyl group having 6 to 22 carbon atoms, the remainder representing H;
(iii) compounds represented by the above formula (I) , wherein one of B1, B2 and B3 represents an acyl group having 6 to 22 carbon atoms; the remainder representing H;
(iv) compounds represented by the following formula (I), wherein each of B1, B2 and B3 represent H;
the weight ratio of the compounds (i) / (ii) / (iii) being 1 to 15 / 9 to 35 / 46 to 90.

These compounds are preferably prepared by a reaction between triglyceride and glycerine and ethylene oxide. The preparation of these compounds is described in detail in the European Patent EP 0 586 323.B1 and in the European Patent Application No. 99 106 233.2.

The acyl group having 6 to 22 carbon atoms, desirably 12 to 18 carbon atoms, is preferably derived from a natural fat or oil or a synthetic glyceride. Preferred fats and oils include vegetable palm kernel oil, sunflower oil, rapeseed oil, castor oil, olive oil, soybean oil; and animal fat such as tallow, bone oil; fish oil, hardened oils and semihardened oils thereof; and mixtures thereof. Particularly preferred are acyl groups derived from coconut oil, palm oil and tallow such as beef tallow.

A particularly preferred ethoxylated glyceride comprises glycereth-17 cocoate, marketed under the trade name Levenol C-201 by Kao S.A. This is a mixture of compounds of the above formula (I) wherein the sum of m, n, and 1 is 17 and either one or two groups of B1 and B2 are acyl groups derived from coconut oil.

The ethoxylated glyceride is used in a weight ratio of ethoxylated glyceride to silicone oil in the range of from 1:1 to 10:1, preferably 2:1 to 8:1. The amount of ethoxylated glyceride is preferably 2 to 10 wt.%, more preferably 5 to 8 wt.% with respect to the total weight of the composition.

The anionic surfactant, component (c), is preferably sodium lauryl ether sulfate, preferably having an average degree of ethoxylation of 1 to 3, more preferably 1 to 2.5, most preferably 2 to 2.5. The anionic surfactant is desirably contained in the composition in an amount of 3 to 15 wt.%, preferably 6 to 15 wt.%.

The weight ratio of component (b) to component (c) is within the range of 1:4 to 4:1, preferably 1:2 to 2:1, most preferably in the range of 6:7 to 7:6.

The total amounts of components (b) and (c) is within the range of 10 to 25 wt.% with respect to the total weight of the composition, preferably within the range of 12 to 20 wt.%. The total amount of wash active matter, that is, the total amounts of surfactants, contained in the composition of the present invention is preferably less than 25 wt.%. That is, if the composition contains surfactants other than components (b) and (c), the total amount of these surfactants and components (b) and (c) does desirably not exceed 25 wt.%.

The compositions of the present invention are optically transparent. According to the present invention, the term "optically transparent" means that the transmission of the composition in the visible region is at least 95%. The compositions of the present invention have preferably a transmission of more than 97 %. The transmission is measured according to DIN 53995 using the Dr.Lange Liquid Tester LTM1 (supplied by Dr. Bruno Lange GmbH&Co. KG, Düsseldorf, Deutschland).

The viscosity of the composition of the present invention is preferably at least 1500 mPa·s, more preferably 2000-3000 mPa·s. The viscosity values indicated in the present invention are measured at 20°C with a Brookfield viscosimeter LVT (supplied by Brookfield Engineering Laboratories Inc. Stoughton, MA, USA) in accordance with DIN 1341 (spindle 2 at 30 rpm for viscosities in the range of up to 1000 mPa; spindle 3 at 12 rpm for viscosities in the range of 1000 to 7000 mPa; spindle 4 at 12 rpm for viscosities in the range of more than 7000 mPa).

The pH value of the composition of the present invention is preferably within the range of 5 to 8, more preferably 6 to 7.

The composition of the present invention may contain other surfactants such as non-ionic surfactants, cationic surfactants, and in particular amphoteric surfactants. Amphoteric surfactant include ampholytes and betaines. Specific examples are alkyl amine oxides, alkyl betaines, alkyl sulphobetaines (sultaines), alkyl glycinates, alkyl carboxyglycinates, alkyl amphoacetates, alkyl amphopropionates, alklyamphoglycinates, alkyl amidopropyl betaines, alkyl amidopropyl- and hydroxysultaines. Particularly preferred amphoteric surfactants are alkyl sulphobetaines (sultaines), alklyamphoglycinates and alkyl amphoacetates. Even more preferred are alkyl hydroxysultaines, in particular lauryl hydroxysultaine. Amphoteric surfactants are preferably present in a weight ratio of ethoxylated glyceride to amphoteric surfactant in the range of 1:3 to 3:1. The total amount of amphoteric surfactant is preferably between 4 and 8 wt.% with respect to the total amount of the composition.

The composition of the present invention may optionally contain fatty alcohols having 6 to 22 carbon atoms.

The composition of the present applications may also contain deposition polymers. Suitable deposition polymers are any which enhance deposition of the silicone oil on the intented site, i.e. the hair or the skin. The deposition polymers disclosed in EP-B-529 883 are preferably used.

The composition of the present invention preferably also contains a vegetable oil. According to the present invention, the term vegetable oil means a mixture of saturated or unsaturated fatty acids having 6 to 22 carbon atoms, triglycerides thereof, esters thereof with alcohols having 6 to 22 carbon atoms, and the corresponding fatty alcohols having 6 to 22 carbon atoms. The vegetable oil may also be a terpinene-containing oil. Preferred examples of the vegetable oils to be used according to the present invention include evening prime rose oil, sesame oil and preferably jojoba oil, macademia nut oil, tea tree oil, and avocado oil.

The vegetable oil is preferably contained in the composition of the present invention in a weight ratio of vegetable oil to silicone oil of 1:3 to 3:1, more preferably 1:1. The total amount of silicone oil and vegetable oil is preferably in the range of 2 to 6 wt.% with respect to the total weight of the composition.

If vegetable oil is present in the composition of the present invention, the hydrophobic silicone oil used is preferably a volatile hydrophobic silicone oil. Volatile hydrophobic silicone oils are silicone oils which evaporate from the hair surface at atmospheric pressure and room temperature.

The weight ratio of ethoxylated glyceride to the total amount of silicone oil and vegetable oil is preferably in the range of 1:1 to 6:1, preferably 2:1 to 4:1.

The composition of the present invention may optionally contain further ingredients such as perfume, preservatives, thickeners, salts, and medically effective substances.

The preparation of the compositions of the present invention often requires a specific preparation method. By simply mixing the ingredients in any arbitrary order, the composition of the present invention may not always be obtained. This method will now be explained in more detail.

The method for preparing the compounds of the present invention comprises the steps:
(a) mixing silicone oil with ethoxylated glyceride derived from carboxylic acids having 6 to 22 carbon atoms in a weight ratio of ethoxylated glyceride to silicone oil in the range of 1:1 to 10:1 at a temperature of 20°C or less;
(b) adding anionic surfactant and stirring until a transparent composition is obtained.

Step (a) is generally carried out under gentle stirring such that incorporation of air into the mixture is minimized. For example, the mixture may simply be hand shaked or preferably stirred using a magnetic stirrer such as IKAMAG (supplied by Janke & Kunkel, Germany). When the mixture has a volume of less than 100 ml, the mixture is preferably stirred at less than 400 RPM, more preferably less than 200 RPM. Low stirring energies are not only advantageous in view of energy costs, but particularly in view of the fact that less air is incorporated into the solution. Air is generally difficult to remove afterwards from the aqueous solution once incorporated and may cause stability problems.

In the following step (b), the anionic surfactant such as sodium lauryl ether sulfate is added and the resulting mixture is stirred until an optically transparent composition is obtained. The anionic surfactant is generally added in diluted form as aqueous solution in a concentration such that gelation is avoided in step (b). The concentration should preferably not exceed 30 wt.-%. Again, it is preferably only gently stirred in step (b).

In a further step (c), subsequent to step (b), the viscosity and the pH of the composition are preferably adjusted to the values indicated above. The viscosity of the composition prior to step (c) depends on the components used. If the viscosity is found to be insufficient, e.g., below 1500 mPa·s, thickeners such as non-ionic surfactant-type thickeners such as Aminol N, cocamide DEA and cocamide MEA and derivatives thereof or polymeric thickeners such as PEG-150 distearate, PEG-120 methyl glucose dioleate, or PEG-160 sorbitan isostearate may be added. However, the amount of polymeric thickeners should preferably not exceed an amount of 1 wt.-% with respect to the total weight of the composition. Higher amounts of polymeric thickener may cause an unpleasant sticky feeling on skin during application.

The pH value may be adjusted to the range of 5 to 8 by adding pH adjusting agents known in the field. Examples for pH adjusting agents include citric acid and NaOH.

In the case that amphoteric surfactants are used as (co-)surfactants, they are preferably added after step (a) and prior to step(b). Further ingredients such as perfume and preservatives are usually added after step (c).

The compositions of the present invention show a number of beneficial properties in view of their high silicone content, and they may not only be used as personal care products such as shampoos, hair conditioners, hair dying agents, levelling agents, shower baths, liquid soaps and other cosmetic rinse-off products, but also in textile applications (softener) and plastic applications (plastic additives). The compositions are particularly useful as hair gloss shampoos, detangling shampoos, silky hair shampoos, fast drying shampoos, elderly people shampoos, colour care shampoos, special care shampoos.

### Examples

In the Examples, all products used were obtained from Kao Chemicals Europe, unless indicated otherwise.

### Example 1: Hair and scalp care shampoo for elderly people

### SHAMPOO RECIPE RECIPE ( ≈ 25 % wash active matter; ≈ 29 % total active matter):

(1) 25 % EMAL 228D ( 28 % a.m.) ( INCI : Sodium Laureth Sulfate )
(2) 18.5 % BETADET S-20 ( 38 % a.m. ) ( INCI: Lauryl Hydroxysultaine )
(3) 6 % AMINOL N ( 91 % a.m. ) ( INCI: PEG-4 Rapeseedamide )
(4) 6 % LEVENOL C-201 ( 100 % a.m. ) ( INCI: Glycereth-17 Cocoate )
(5) 1.5 % Cyclomethicone ( IUPAC Decamethylcyclopentasiloxane, supplied by Dow Corning )
(6) 1.5 % Jojoba oil
(7) 0.7 % RHEODOL TW-IS399C ( 100 % a.m.) ( INCI: PEG-160 Sorbitan Isostearate ) q.s. ad 100 % : water, citric acid, perfume, preservative

### SHAMPOO PREPARATION:

- (5) and (6) are stirred briefly ( ≈ 2 minutes ) for intermixing resulting in a clear liquid
- (4) is added and mixture is stirred briefly ( ≈ 3 minutes ) for intermixing resulting in a turbid liquid
- (3) is added and stirred briefly ( ≈ 4 minutes ) resulting in a clear liquid
- (2) is added and stirred briefly ( ≈ 3 minutes ) resulting in a clear liquid of higher viscosity
- (1) is dissolved in 50 % of the water and added together with preservative to above mixture and stirred for ≈ 3 minutes resulting in a clear liquid of lower viscosity
- (7) is dissolved in-rest of the water at 50 °C & added together with perfume to above mixture and stirred for ≈ 8 minutes resulting in a clear liquid of suitable viscosity (≈1600 mPas at 20 °C)
- pH-value is adjusted by citric acid ( pH : 6 - 7 )

### Example 2: Hair gloss shampoo

### SHAMPOO RECIPE RECIPE ( ≈ 23 % wash active matter; ≈ 26 % total active matter ):

(1) 25 % EMAL 228D (28 % a.m.) (INCI: Sodium Laureth Sulfate)
(2) 18.5 % BETADET S 20 ( 38 % a.m. ) ( INCI: Lauryl Hydroxysultaine )
(3) 6 % LEVENOL C-201 ( 100 % a.m. ) ( INCI: Glycereth-17 Cocoate )
(4) 1.5 % Phenyl Trimethicone (supplied by Dow Corning )
(5) 0.5 % TETRANYL CO-40 ( 80 % a.m.) ( INCI : Dioleoylethyl Hydroxyethylmonium Methosulfate)
(6) 3 % AMINOL N ( 91 % a.m.) (INCI: PEG-4 Rapeseed amide)
(7) 0.7 % RHEODOL TW-IS399C (100 % a.m.) ( INCI: PEG-160 Sorbitan Isostearate ) q.s. ad 100 % : water, sodium hydroxide, perfume, preservative

### SHAMPOO PREPARATION:

- (5) is dissolved in (4) and stirred briefly ( ≈ 3 minutes.) for intermixing, resulting in a clear mixture
- (3) is added and stirred for ≈ 3 minutes, resulting in a clear liquid
- (2) is added and stirred for ≈ 2 minutes resulting in a more viscous, turbid liquid
- after addition of (6) and stirring for ≈ 2 minutes, liquid becomes clear and less viscous again
- (1) is dissolved in 50 % of the water and added together with preservative to above mixture and stirred for ≈ 3 minutes resulting in a clear liquid of lower viscosity
- (7) is dissolved in rest of the water at 50 °C and added together with the perfume to above mixture and stirred for ≈ 8 minutes resulting in a clear liquid of suitable viscosity (≈1930 mPas at 20 °C)
- pH-value is adjusted by NaOH (50 % a.m.)

## Claims

1. An optically transparent aqueous composition comprising
(a) a hydrophobic silicone oil in an amount of 1-3 wt.-% with respect to the total weight of the composition;
(b) ethoxylated glycerides derived from carboxylic acids having 6 to 22 carbon atoms; and
(C) an anionic surfactant;
wherein the weight ratio of component (b) to component (a) is in the range of 1:1 to 10:1; and wherein the total amount of the components (b) and (c) is in the range of 10-25 wt.-% with respect to the total weight of the composition.

2. The aqueous composition according to claim 1, wherein the ethoxylated glyceride derived from carboxylic acids having 6 to 22 carbon atoms comprises glycereth-17 cocoate.

3. The aqueous composition according to any of the preceding claims, wherein the hydrophobic silicone oil is a non-volatile silicone oil.

4. The aqueous composition according to any of the preceding claims, additionally containing a vegetable oil.

5. The aqueous composition according to claim 4, wherein the hydrophobic silicone oil is a volatile silicone oil.

6. The aqueous composition according to any of the preceding claims, additionally containing an amphoteric surfactant.

7. The aqueous composition according to claim 6, wherein the amphoteric surfactant is lauryl hydroxysultaine.

8. The aqueous composition according to claim 6 and 7, wherein the amphoteric surfactant is present in an amount of 4 to 8 wt.-% with respect to the total weight of the composition.

9. The aqueous composition according to any of the preceding claims, wherein the anionic surfactant is sodium lauryl ether sulfate having an average degree of ethoxylation in the range of 1 to 3.

10. The aqueous composition according to any of the preceding claims, wherein the composition has a viscosity of at least 1500 mPa s.

11. The aqueous composition according to any of the preceding claims, wherein the composition has a pH value in the range of 5 to 8.

12. Method for preparing a composition according to claim 1, comprising the steps of:
(a) mixing silicone oil with ethoxylated glyceride derived from carboxylic acids having 6 to 22 carbon atoms in a weight ratio of ethoxylated glyceride to silicone oil in the range of 1:1 to 10:1; and
(b) adding anionic surfactant and stirring until a transparent composition is obtained.

13. Use of the composition of any of claims 1 to 11 as a hair shampoo.

## Patentansprüche

1. Optisch transparente, wässrige Zusammensetzung, umfassend:
(a) ein hydrophobes Siliconöl in einer Menge von 1 bis 3 Gew.% in bezug auf das Gesamtgewicht der Zusammensetzung;
(b) ethoxylierte Glyceride, die sich von Carbonsäuren mit 6 bis 22 Kohlenstoffatomen ableiten; und
(c) ein anionisches Tensid;
wobei das Gewichtsverhältnis von Komponente (b) zu Komponente (a) im Bereich von 1:1 bis 10:1 liegt; und wobei die Gesamtmenge der Komponenten (b) und (c) im Bereich von 10 bis 25 Gew.% in bezug auf das Gesamtgewicht der Zusammensetzung liegt.

2. Wässrige Zusammensetzung gemäss Anspruch 1, wobei das ethoxylierte Glycerid, das sich von Carbonsäuren mit 6 bis 22 Kohlenstoffatomen ableitet, Glycereth-17-cocoat umfasst.

3. Wässrige Zusammensetzung gemäss einem der vorhergehenden Ansprüche, wobei das hydrophobe Siliconöl ein nicht-flüchtiges Siliconöl ist.

4. Wässrige Zusammensetzung gemäss einem der vorhergehenden Ansprüche, weiterhin enthaltend ein pflanzliches Öl.

5. Wässrige Zusammensetzung gemäss Anspruch 4, wobei das hydrophobe Siliconöl ein flüchtiges Siliconöl ist.

6. Wässrige Zusammensetzung gemäss einem der vorhergehenden Ansprüche, weiterhin enthaltend ein amphoteres Tensid.

7. Wässrige Zusammensetzung gemäss Anspruch 6, wobei das amphotere Tensid Laurylhydroxysultain ist.

8. Wässrige Zusammensetzung gemäss Anspruch 6 und 7, wobei das amphotere Tensid in einer Menge von 4 bis 8 Gew.% in bezug auf das Gesamtgewicht der Zusammensetzung vorliegt.

9. Wässrige Zusammensetzung gemäss einem der vorhergehenden Ansprüche, wobei das anionische Tensid Natriumlaurylethersulfat mit einem durchschnittlichen Ethoxylierungsgrad im Bereich von 1 bis 3 ist.

10. Wässrige Zusammensetzung gemäss einem der vorhergehenden Ansprüche, wobei die Zusammensetzung eine Viskosität von mindestens 1.500 mPa·s aufweist.

11. Wässrige Zusammensetzung gemäss einem der vorhergehenden Ansprüche, wobei die Zusammensetzung einen pH-Wert im Bereich von 5 bis 8 aufweist.

12. Verfahren zur Herstellung einer Zusammensetzung gemäss Anspruch 1, umfassend die Schritte
(a) Mischen des Siliconöls mit ethoxyliertem Glycerid, abgeleitet von Carbonsäuren mit 6 bis 22 Kohlenstoffatomen, in einem Gewichtsverhältnis von ethoxyliertem Glycerid zu Siliconöl im Bereich von 1:1 bis 10:1; und
(b) Zugabe von anionischem Tensid und Rühren, bis eine transparente Zusammensetzung erhalten wird.

13. Verwendung der Zusammensetzung gemäss einem der Ansprüche 1 bis 11 als Haarshampoo.

## Revendications

1. Composition aqueuse optiquement transparente comprenant :
(a) une huile de silicone hydrophobe à raison de 1 à 3 % en poids par rapport au poids total de la composition ;
(b) des glycérides éthoxylés dérivés d'acides carboxyliques ayant de 6 à 22 atomes de carbone ; et
(c) un tensioactif anionique ;
dans laquelle le rapport pondéral du composant (b) au composant (a) se situe dans la gamme de 1:1 à 10:1 et dans laquelle la quantité totale des composants (b) et (c) se situe dans la gamme de 10 à 25% en poids par rapport au poids total de la composition.

2. Composition aqueuse selon la revendication 1, dans laquelle le glycéride éthoxylé dérivé d'acides carboxyliques ayant de 6 à 22 atomes de carbone comprend le glycereth-17 cocoate.

3. Composition aqueuse selon l'une quelconque des revendications précédentes, dans laquelle l'huile de silicone hydrophobe est une huile de silicone non volatile.

4. Composition aqueuse selon l'une quelconque des revendications précédentes, contenant, en outre, une huile végétale.

5. Composition aqueuse selon la revendication 4, dans laquelle l'huile de silicone hydrophobe est une huile de silicone volatile.

6. Composition aqueuse selon l'une quelconque des revendications précédentes, contenant, en outre, un tensioactif amphotère.

7. Composition aqueuse selon la revendication 6, dans laquelle le tensioactif amphotère est la lauryl hydroxysultaine.

8. Composition aqueuse selon les revendications 6 et 7, dans laquelle le tensioactif amphotère est présent à raison de 4 à 8% en poids par rapport au poids total de la composition.

9. Composition aqueuse selon l'une quelconque des revendications précédentes, dans laquelle le tensioactif anionique est le lauryl éther sulfate de sodium ayant un degré moyen d'éthoxylation dans la gamme de 1 à 3.

10. Composition aqueuse selon l'une quelconque des revendications précédentes, dans laquelle la composition a une viscosité d'au moins 1500 mPa s.

11. Composition aqueuse selon l'une quelconque des revendications précédentes, dans laquelle la composition a un pH dans la gamme de 5 à 8.

12. Procédé pour la préparation d'une composition selon la revendication 1, comprenant les étapes de :
(a) mélange d'une huile de silicone avec un glycéride éthoxylé dérivé d'acides carboxyliques ayant 6 à 22 atomes de carbone dans un rapport pondéral du glycéride éthoxylé à l'huile de silicone dans la gamme de 1:1 à 10:1 et
(b) addition du tensioactif anionique et agitation jusqu'à l'obtention d'une composition transparente.

13. Utilisation de la composition selon l'une quelconque des revendications 1 à 11 comme shampoing pour cheveux.
